# EUROPÄISCHE PATENTSCHRIFT

(11) **EP 3 730 219 B1**
(45) Veröffentlichungstag und Bekanntmachung des Hinweises auf die Patenterteilung: **23.03.2022**
(21) Anmeldenummer: 19171383.3
(22) Anmeldetag: 26.04.2019
(51) Int. Cl.: B05B 11/00

(54) **SPENDER ZUM AUSTRAG PHARMAZEUTISCHER FLÜSSIGKEITEN**
DISPENSER FOR DISCHARGING LIQUIDS
DISTRIBUTEUR DESTINÉ À LA SORTIE DES LIQUIDES PHARMACEUTIQUES

(43) Veröffentlichungstag der Anmeldung: 28.10.2020
(73) Patentinhaber: Aptar Radolfzell GmbH, 78315 Radolfzell (DE)
(72) Erfinder: Greiner-Perth, Jürgen, 78244 Gottmadingen (DE); Herz, Andi, 78253 Eigeltingen-Reute (DE)
(74) Vertreter: Witte, Weller und Partner Patentanwälte mbB Stuttgart

(56) Entgegenhaltungen:
- EP-A2- 1 170 061
- DE-T2- 60 009 058
- GB-A- 2 198 038
- US-A1- 2006 065 677
- US-A1- 2007 102 455
- US-A1- 2009 008 415
- US-A1- 2011 250 007
- US-A1- 2013 218 066
- US-A1- 2014 371 690

## Beschreibung

### ANWENDUNGSGEBIET UND STAND DER TECHNIK

Die Erfindung betrifft einen Spender zum Austrag pharmazeutischer Flüssigkeiten, insbesondere für den tropfenweisen Austrag von pharmazeutischen Flüssigkeiten in Form von Einzeltropfen.

Ein gattungsgemäßer Spender weist einen Flüssigkeitsspeicher, eine Pumpeinrichtung mit einer volumetrisch veränderlichen Pumpkammer sowie eine Abgabeöffnung zur Abgabe der Flüssigkeit auf. Mittels der Pumpeinrichtung kann Flüssigkeit zur Abgabeöffnung gefördert werden. Der Spender ist als so genannter Side-Actuation-Spender ausgebildet. Hierunter ist zu verstehen, dass der Spender ein entlang einer Haupterstreckungsrichtung ausgerichtetes längliches Gehäuse aufweist und an einem distalen Ende des Spenders die Abgabeöffnung vorgesehen ist, wobei ein Betätigungstaster zur Betätigung der Pumpeinrichtung im Bereich einer Mantelfläche des Gehäuses seitlich am Spender vorgesehen ist. Die Handhabung erfolgt üblicherweise derart, dass die Mantelfläche des Gehäuses umgriffen wird und die Betätigung des Betätigungstasters mit dem Daumen stattfindet.

Aus der DE 102006012898 A1 ist bereits ein gattungsgemäßer Side-Actuation-Spender bekannt, der als Tropfenspender ausgebildet ist. Einige Aspekte der dort beschriebenen Spender sind jedoch nachteilig. So kann es dem Benutzer schwer fallen, bei Betätigung mit dem Daumen die Verlagerung der dortigen Betätigungshandhabe dosiert einzudrücken. Auch ist die Inbetriebnahme dadurch erschwert, dass sich bei Auslieferung zu viel Luft in der Pumpkammer und dem Austragkanal befindet und das Abgabeventil daher bereits bei sehr geringem Überdruck öffnen muss, um das Austreiben der Luft durch initiale Betätigung zu ermöglichen. Des Weiteren bedürfen gerade Tropfenspenders einer sehr genauen Abstimmung aller fluidführenden Komponenten, was es schwer macht, einen solchen Spender nach Kundenwünschen anzupassen, also insbesondere an eine markentypische Gestaltung eines Herstellers von pharmazeutischen Flüssigkeiten in Hinblick auf Form und Farbe anzupassen. Die US2014/371690 A1 zeigt ein Abgabesystem nach dem Stand der Technik.

### AUFGABE UND LÖSUNG

Aufgabe der Erfindung ist es, einen beschriebenen gattungsgemäßen Side-Actuation-Spender dahingehend weiterzubilden, dass die beschriebenen Nachteile überwunden oder zumindest verringert werden.

Zur Lösung der Aufgabe wird ein Spender vorgeschlagen, der einen Flüssigkeitsspeicher zur Lagerung der Flüssigkeit vor dem Austrag, ein Gehäuse mit einer darin integrierten Pumpeinrichtung mit einer volumetrisch veränderlichen Pumpkammer sowie eine Abgabeöffnung zur Abgabe der Flüssigkeit aufweist. Die Pumpeinrichtung dient dem Zweck, Flüssigkeit aus dem Flüssigkeitsspeicher anzusaugen und bei einer Betätigung des Spenders durch die Abgabeöffnung abzugeben.

Der Spender ist als Side-Actuation-Spender ausgestaltet und weist daher ein in einer Haupterstreckungsrichtung ausgerichtetes längliches Gehäuse und an einem distalen Ende des Spenders eine Abgabeöffnung auf. Das Gehäuse kann einen im Wesentlichen runden Querschnitt aufweisen, kann aber auch hiervon abweichen und eine eher flache Bauform aufweisen. Der Flüssigkeitsspeicher ist vorzugsweise an dem der Abgabeöffnung gegenüber liegenden Ende vorgesehen. Ein Betätigungstaster zur Betätigung der Pumpeinrichtung ist im Bereich einer Mantelfläche des Gehäuses seitlich am Spender vorgesehen und zwischen einer unbetätigten Ausgangslage und einer in Richtung einer Mittelachse eingedrückten betätigten Endlage verlagerbar. Die Betätigung des Betätigungstasters erfolgt dabei üblicherweise mittels des Daumens.

Die Pumpeinrichtung kann insbesondere mit einer abschnittsweise verformbaren Pumpkammerwandung, beispielsweise realisiert durch ein in sich verformbares Membranteil, oder einer als Ganzes verlagerbaren Pumpkammer in Art eines Kolbens versehen sein. Das Eindrücken des Betätigungstasters bewirkt die Verkleinerung der Pumpkammer, insbesondere indem der Kolben oder das Membranteil quer zur Haupterstreckungsrichtung verlagert wird. Üblicherweise ist die Pumpkammer durch zwei Ventile begrenzt, nämlich eingangsseitigdurch ein Einlassventil, welches bei einem Unterdruck in der Pumpkammer gegenüber dem Flüssigkeitsspeicher öffnet, sowie ausgangsseitig durch ein Auslassventil, welches bei einem Überdruck in der Pumpkammer gegenüber einem zur Abgabeöffnung führenden Abgabekanal öffnet. Das Einlassventil ist bei identischem Druck im Flüssigkeitsspeicher und in der Pumpkammer geschlossen und öffnet vorzugsweise ab einem Unterdruck von etwa 0,1 bis 0,2 bar in der Pumpkammer. Das Auslassventil ist bei identischem Druck in der Pumpkammer und dem sich anschließenden Abgabekanal geschlossen und öffnet vorzugsweise ab einem Überdruck von etwa 0,5 bar in der Pumpkammer. Vorzugsweise ist der Abgabeöffnung ein weiteres Ventil vorgeschaltet, welches bei einem Überdruck der Flüssigkeit im Abgabekanal gegenüber einem Umgebungsdruck öffnet.

Die nachfolgend beschriebenen Aspekte der Erfindung sind insbesondere bei der Realisierung eines Tropfenspenders von Vorteil, also eines Spenders, mit dem bestimmungsgemäß pharmazeutische Flüssigkeit in Form einzelner Tropfen abgegeben wird. Hierfür ist vorzugsweise jenseits der Abgabeöffnung ein Tropfenbildungsmittel vorgesehen, also ein Bereich, in dem die abgegebenen Flüssigkeit sich sammelt, bis das abgegebene Volumen reicht, um einen Tropfen zu bilden, der sich schwerkraftbedingt von den Tropfenbildungsmitteln löst. Insbesondere kann es sich bei den Tropfenbildungsmitteln um eine die Abgabeöffnung umgebende Tropfenbildungsfläche handeln, die insbesondere eben oder konkav geformt und/oder außenseitig durch eine scharfkantige Abrisskante mit einem Krümmungsradius von vorzugsweise weniger als 0,5mm begrenzt ist. Das genannte Abgabeventil ist bei einem Tropfenspender üblicherweise dafür ausgebildet, bei Überdruck von vorzugsweise mindestens 0,3 bar, vorzugsweise mindestens 0,5 bar zu öffnen, damit die Flüssigkeit mit nur geringem Druck die Abgabeöffnung durchströmt und jenseits dieser sich zunächst an den genannten Tropfenbildungsmitteln sammelt. Der Überdruck zum Öffnen des Ventils sollte bei pharmazeutischen Flüssigkeiten ohne Konservierungsmittel nicht zu gering sein, um möglichst zu verhindern, dass externe Druckschwankungen ein Öffnen bewirken. Bei Flüssigkeiten mit Konservierungsmittel sind die Anforderungen geringer. Hier können auch Öffnungsdrücke des Abgabeventils hinab bis 0,05 bar möglich und vorteilhaft sein.

Sofern ein erfindungsgemäßer Spender als Tropfenspender ausgestaltet ist, weist die Pumpkammer vorzugsweise ein nutzbares Volumen, gebildet durch die Volumendifferenz zwischen Maximalvolumen und Minimalvolumen der Pumpkammer, auf, welches zwischen 20 µl und 50 µl, vorzugsweise zwischen 30 µl und 40 µl, beträgt. Ein solches Volumen entspricht in etwa dem Volumen eines Tropfens bei üblichen Gestaltungen von Tropfenbildungsmitteln. Der Benutzer kann somit durch eine Betätigung des Betätigungstasters genau einen Tropfen ausbringen. Sofern die Tropfenbildungsmittel zur Bildung von größeren Tropfen ausgebildet sind, ist das nutzbare Volumen der Pumpkammer vorzugsweise entsprechend größer. Auch bei der Gestaltung des Spenders als Tropfenspender kann eine größere Pumpkammer dann zweckmäßig sein, wenn mit einer Betätigung mehrere Tropfen abgeben werden sollen. Ein Quotient zwischen einem minimalen Pumpkammervolumen und einem maximalen Pumpkammervolumen beträgt vorzugsweise maximal 1:2, insbesondere vorzugsweise maximal 1:3. Hierdurch wird das initiale Austreiben von Luft aus der Pumpkammer zum Zwecke des nachfolgenden Ansaugens von Flüssigkeit aus dem Flüssigkeitsspeicher erleichtert.

Die Tropfenbildungsmittel und das nutzbare Pumpkammervolumen müssen nicht zwingend derart abgestimmt sein, dass das Pumpkammervolumen zur Bildung mindestens eines schwerkraftbedingt ablösbaren Tropfens ausreicht. So kann beispielsweise auch ein geringeres Pumpkammervolumen als 20 µl vorgesehen sein, wenn eine andere Art der Ablösung vorgesehen ist, insbesondere ein Ablösung unter Einwirkung eines Druckstoßes. Ein solcher Druckstoß kann beispielsweise dadurch erzielt werden, dass bei Betätigung eine erzwungene Mindestkraft aufgebracht werden muss, wie im weiteren noch erläutert ist.

Gemäß einem ersten Aspekt der Erfindung ist vorgesehen, dass die volumetrisch veränderliche Pumpkammer an einer Seite durch eine verlagerbare und/oder verformbare Pumpkammerwandung begrenzt ist, die mit dem Betätigungstaster in einem Verbindungsbereich derart verbunden ist, dass sie die Pumpkammerwandung durch Eindrücken des Betätigungstasters in einer Verlagerungsrichtung etwa orthogonal zur Haupterstreckungsrichtung in Richtung der Pumpkammer verlagerbar ist und hierbei die Pumpkammerwandung verlagert.

Der Betätigungstaster selbst ist gemäß diesem ersten Aspekt der Erfindung als Kipptaster ausgebildet, der um eine Kippachse kippbeweglich gegenüber dem Gehäuse gelagert ist. Die Kippachse ist dabei vorzugsweise orthogonal zur Haupterstreckungsrichtung ausgerichtet. Der Betätigungstaster wird vom Benutzer zur Betätigung der Pumpeinrichtung eingedrückt. Zum Zwecke der Rückstellung des Betätigungstasters in seine unbetätigte Ausgangslage weist der Spender eine Federeinrichtung auf, die in einem Krafteinkopplungsbereich auf den Betätigungstaster wirkt und diesen in Richtung der Ausgangslage zurückdrückt. Vorzugsweise ist der Kipptaster derart gelagert, dass er um mindestens 5° verkippbar ist, insbesondere vorzugsweise um mindestens 10°.

Die Verwendung eines Kipptasters gestattet eine recht genaue Dosierung. Dies hängt vor allem damit zusammen, dass der Kipptaster durch seine schwenkbare Anlenkung nicht zum Verkanten oder dergleichen neigt und dass der Benutzer durch den Abstand der manuellen Kraftbeaufschlagung von der Kippachse gut steuern kann, mit welcher Geschwindigkeit der Kipptaster eingedrückt wird. Dies kann insbesondere bei der Tropfenabgabe von Vorteil sein, da hierdurch der Ablösezeitpunkt des Tropfens von den Tropfenbildungsmitteln genau beeinflussbar ist.

Die Federeinrichtung, die auf den Kipptaster eine rückstellende Kraft ausübt, kann grundsätzlich als einstückig mit dem Kipptaster und/oder einstückig mit dem Gehäuse ausgebildeter elastisch verformbarer Federabschnitt ausgebildet sein oder nicht erfindungsgemäß durch eine in sich verformbare Pumpkammerwandung oder ein hieran angeformtes Federsegment gebildet werden. Bevorzugt wird allerdings eine separate Federeinrichtung, insbesondere in Form einer Schraubenfeder aus Kunststoff oder Metall. Die Federeinrichtung drückt den Betätigungstaster in seiner unbetätigten Ausgangslage, wobei hierbei die Pumpkammer wieder vergrößert wird und dabei Flüssigkeit aus dem Flüssigkeitsspeicher ansaugt.

Die Kippachse des Kipptasters ist vorzugsweise bezogen auf die Haupterstreckungsrichtung an einem von der Abgabeöffnung weg weisenden Ende des Kipptasters vorgesehen. Dies gestattet es bei üblichem Umgreifen des Gehäuses und Auflegen des Daumens auf den Kipptaster, dass der Daumen während der Betätigung dauerhaft flächig auf dem Kipptaster aufliegt und somit die Kippbewegung besonders feinfühlig steuerbar ist.

Je nach Anwendungszweck kann es gewünscht sein, eine Mindestkraft zur Betätigung zu gewährleisten. Dies kann durch einen Kipptaster in besonders einfacher Form erfolgen, nämlich dadurch, dass auf der der Kippachse abgewandten Seite des Kipptasters eine Raste vorgesehen ist, die ein Eindrücken des Kipptasters erschwert. Vorzugsweise sind diese Raste und der Kipptasters derart aufeinander abgestimmt, dass es einschließlich der durch die Federeinrichtung bewirkten entgegengesetzten Kraft einer Betätigungskraft von 10 Newton am distalen Ende des Betätigungstasters bedarf, um die Raste zu überwinden. Vorzugsweise liegt die erforderliche Betätigungskraft noch höher, insbesondere bei mindestens 20 Newton. Durch eine geeignete Geometrie der Raste kann bewirkt werden, dass die Rückführung die Ausgangslage einschließlich der Überwindung der Raste dennoch alleine von der Kraft der Federeinrichtung geleistet werden kann.

Die Verwendung einer Raste ist insbesondere zweckmäßig, um einen Druckstoß zu erzeugen, der das Ablösen kleiner Tropfen von den Tropfenbildungsmitteln gestattet, die sich rein schwerkraftbedingt nicht lösen würden. Der Druckstoß kann durch die mittels der Raste erzwungene Mindestkraft bewirkt werden.

Wenn eine geringe Länge des Spenders und eine dadurch bedingte kurze Ausgestaltung des Kipptasters in Haupterstreckungsrichtung es nicht anderweitig gestattet oder andere hierfür sprechende Gründe vorliegen, kann nicht erfindungsgemäß vorgesehen sein, dass derVerbindungsbereich und der Krafteinkopplungsbereich im Wesentlichen übereinstimmen. Dies ist beispielsweise gegeben, wenn der Anbringungsort eines Kolbens oder eines Membranteils am Kipptaster von einer als Schraubenfeder ausgestalteten Feder umgeben ist.

Erfindungsgemäß vorgesehen ist es allerdings, wenn der Krafteinkopplungsbereich und der Verbindungsbereich signifikant unterschiedlich weit von der Kippachse entfernt am Kipptaster vorgesehen sind, wobei insbesondere vorzugsweise der Abstand zwischen der Kippachse und dem Verbindungsbereich geringer ist als der Abstand zwischen der Kippachse und dem Krafteinkopplungsbereich. Voneinander abweichende Abstände des Verbindungsbereichs und des Krafteinkopplungsbereichs zur Kippachse weisen verschiedene Vorteile auf, insbesondere auch die einfachere Montage, da die Rückstellfeder und die Verbindung mit der verlagerbaren Pumpkammerwandung örtlich voneinander getrennt sind.

Die Kippbewegung des Kipptasters bewirkt die zumindest partielle Verlagerung der Pumpkammerwandung, wodurch der Druck in der Pumpkammer steigt und Flüssigkeit in Richtung der Abgabeöffnung gedrückt wird. Insbesondere bei Verwendung eines in sich starren Kolbens, der diese Pumpkammer bildet, kann es erforderlich sein, eine Gleitverbindung oder ein Getriebe mit mindestens einem Zwischenelement zwischen dem Betätigungstaster und dem Kolben vorzusehen, um die grundsätzlich rotative Bewegung des Kipptasters in eine lineare Bewegung des Kolbens zu überführen. Da der Kippwinkel jedoch üblicherweise 5° bis 10° nicht übersteigt, ist dies nicht in jedem Fall erforderlich.

Insbesondere kann dieser Bedarf eines Getriebes oder einer Gleitverbindung vermieden werden, indem zwischen dem Kipptaster und der Pumpkammerwandung ein elastisch verformbarer Ausgleichsabschnitt vorgesehen ist, also ein Element aus einem leicht verformbaren Kunststoff, welches zwischen Kipptaster sowie an einem Träger der Pumpkammerwandung angebracht ist. Noch darüber hinaus geht eine Gestaltung, bei der die Pumpkammerwandung durch ein verformbares Membranteil gebildet wird. Ein solches Membranteil kann die Pumpkammer auf einer Seite verschließen, insbesondere auf einer einem Einlassventil und/oder einem Auslassventil gegenüberliegenden Seite. Das Membranteil besteht zumindest teilweise und insbesondere zumindest überwiegend aus einem elastisch verformbaren Material, so dass das Membranteil umlaufend befestigt sein kann und dennoch in die Pumpkammer einrücken kann. Das Membranteil ist vorzugsweise direkt mit dem Kipptaster verbunden und insbesondere vorzugsweise mittels einer Klemmverbindung am Kipptaster befestigt. Der Kipptaster ist um die vorzugsweise orthogonal zur Haupterstreckungsachse ausgerichtete Kippachse schwenkbeweglich. Grundsätzlich kann eine solche Schwenkbeweglichkeit mittels eines einfachen Filmscharniers erzielt werden. Demgegenüber bevorzugt ist es jedoch, wenn am Kipptaster oder an einem umgebenden Gehäuseabschnitt Achsabschnitte vorgesehen sind, die in Lager auf der jeweils anderen Seite eingreifen und hierdurch die Kippbeweglichkeit ermöglichen. Die Lager werden dabei vorzugsweise durch am Kipptaster oder dem Gehäuse einstückig angespritzte Kunststoffbögen mit Durchbrechungen zur Aufnahme eines Achsabschnitts oder Kunststofflaschen mit Vertiefungen zur Aufnahme eines Achsabschnitts gebildet, die insbesondere vorzugsweise mit Montageschrägen versehen sind, um die Achsabschnitte hier orthogonal zur Kippachse hineinzudrücken. Eine derartige Montage ist recht gut automatisierbar.

Ein ähnlicher Aufbau mit angespritzten Kunststoffbögen oder Kunststofflaschen und hierin gesicherten Abschnitten findet sich vorzugsweise auch zur Sicherung des Kipptasters derart, dass eine Ausschwenkbewegung von der Endlage kommend über die Ausgangslage hinaus sicher verhindert wird. Hier sind die Durchbrechungen bzw. die Vertiefungen jedoch entsprechend größer gestaltet, um die mit der Kippbewegung einhergehende Relativverlagerung vom Kipptaster zum Gehäuse zu ermöglichen.

Gemäß einem zweiten Aspekte der Erfindung wird vorgeschlagen, dass bei einem Spender gattungsgemäßer Art der Spender einen in Haupterstreckungsrichtung ausgerichteten Kanalabschnitt aufweist, der stromabwärts der Pumpkammer und insbesondere eines Auslassventils der Pumpeinrichtung vorgesehen ist und durch den Flüssigkeit in Richtung der Abgabeöffnung förderbar ist. Der Spender weist dabei einen Einsatz auf, der vom distalen Ende aus in diesen Kanalabschnitt eingeschoben ist, um dessen freies Volumen zu reduzieren.

Der genannte Kanalabschnitt ergibt sich technisch daraus, dass bei der genannten Side-Actuation-Bauweise und insbesondere bei Verwendung eines Auslassventils, welches auf der der verlagerbaren Pumpkammerwandung gegenüberliegend angeordnet ist, der Flüssigkeitspfad vom Auslassventil bis zur Abgabeöffnung oder dem der Abgabeöffnung vorgeschalteten Abgabeventil recht lang ist. Um diesen Kanalabschnitt in einem inneren Gehäusebauteil aus Kunststoff herstellen und entformen zu können, bedarf es eines vergleichsweisen großen Querschnitts von mindestens etwa 4 mm². Dadurch erhält der Kanalabschnitt jedoch ein recht großes Volumen, was die Inbetriebnahme des Spenders erschwert, da bei der Inbetriebnahme zunächst die Luft aus diesem Kanalabschnitt ausgetrieben werden muss. Daher ist erfindungsgemäß der Einsatz vorgesehen, der das Volumen des Kanalabschnitts reduziert. Vorzugsweise beträgt das im Kanalabschnitt durch den vorzugsweise lanzenartig geformten Einsatz verdrängte Volumen mindestens 30 % des Volumens im Kanalabschnitt ohne eingesetzten Einsatz, insbesondere vorzugsweise mindestens 50 %. Insbesondere vorzugsweise weisen der Kanalabschnitt und der Einsatz einen länglichen Querschnitt auf, der sich vom distalen Ende des Gehäusebauteils stromaufwärts stetig verkürzt.

Der Kanalabschnitt ist vorzugsweise in einem ersten Gehäusebauteil aus Kunststoff vorgesehen, welches auch Wandungen der Pumpkammer und/oder eine Zuleitung zwischen dem Flüssigkeitsspeicher und der Pumpkammer bildet. Der Einsatz ist vorzugsweise Teil eines zweiten Gehäusebauteils aus Kunststoff, das am distalen Ende des ersten Gehäusebauteils angeordnet ist und das von der Abgabeöffnung durchdrungen ist. Diese Zweiteiligkeit ist insbesondere zweckmäßig, um zwischen dem ersten und dem zweiten Gehäusebauteil einen Aufnahmeraum für einen verlagerbaren oder verformbaren Ventilkörper des Abgabeventils zu schaffen. Durch die Gestaltung des zweiten Gehäusebauteils mit dem Einsatz wird eine vereinfachte Montage erzielt, da das zweite Gehäusebauteil gleichzeitig das Auslassbauteil mit der Abgabeöffnung und den Einsatz bildet.

Vorzugsweise weist das erste Gehäusebauteil an seinem distalen Ende einen ersten umlaufenden Steg und das zweite Gehäusebauteil einen zweiten umlaufenden Steg auf, wobei eine Dichtfläche an der Außenseite des zweiten umlaufenden Stegs unter Bildung eines umlaufenden Dichtbereichs an einer Innenseite des ersten umlaufenden Stegs anliegt. In dem hierdurch isolierten Raum kann der genannte Ventilkörper des Abgabeventils angeordnet sein. Der Einsatz, der in den Kanalabschnitt ragt, ist vorzugsweise an dem genannten zweiten und innenliegenden umlaufenden Steg vorgesehen.

Gemäß einem dritten Aspekt der Erfindung, der vom ersten und zweiten Aspekt unabhängig realisiert sein kann, wird vorgeschlagen, dass das Gehäuse zwei Gehäusebauteile aufweist, die bezüglich der grundsätzlichen Funktion den genannten beiden Gehäusebauteilen entsprechen. Das erste Gehäusebauteil begrenzt die Pumpkammer zumindest abschnittsweise. Das zweite, sich daran anschließende Gehäusebauteil ist von der Abgabeöffnung durchdrungen. Weiterhin ist ein hiervon getrenntes Außengehäusebauteil vorgesehen, welches das erste und zweite Gehäusebauteil zumindest teilweise umgibt, wobei das zweite Gehäusebauteil durch eine Durchbrechung des Außengehäusebauteils aus diesem herausragt und wobei eine weitere Durchbrechung im Außengehäusebauteil zur Betätigung des Betätigungstasters vorgesehen ist. Dieses Außengehäusebauteil weist keinerlei flüssigkeitsführende Flächen auf, die einen Flüssigkeitspfad zwischen Flüssigkeitsspeicher und Abgabeöffnung begrenzen.

Das Außengehäusebauteil ist somit funktional nicht unmittelbar relevant für die Führungder Flüssigkeit vom Flüssigkeitsspeicher bis zur Abgabeöffnung. Aus diesem Grund ist seine Bauweise vergleichsweise einfach an unterschiedliche Anforderungen anpassbar, insbesondere in Hinblick auf ästhetische Aspekte und/oder in Hinblick auf die markentypische Ausgestaltung eines Herstellers von pharmazeutischen Flüssigkeiten. Spender der erfindungsgemäßen Art können somit für unterschiedliche Zwecke oder unterschiedliche Kunden unterschiedlich ausgestaltet werden, wobei jedoch das erste und das zweite Gehäusebauteil, die in Hinblick auf die optimale Betätigungs- und Austragcharakteristik optimiert sind, unverändert bleiben.

Das Außengehäusebauteil kann dabei vollständig optional sein, so dass der Spender uneingeschränkt in technischer Hinsicht auch bei entferntem Außengehäusebauteil verwendbar ist. Von Vorteil ist es jedoch, wenn das Außengehäuse eine strukturelle Funktion übernimmt, insbesondere indem das zweite Gehäusebauteil zwischen einem distalen Ende des Außengehäusebauteils einerseits und einem distalen Ende des ersten Gehäusebauteils gesichert ist, vorzugsweise eingeklemmt ist. Das Außengehäusebauteil ist zu diesem Zweck vorzugsweise am ersten Gehäuseteil befestigt.

Von Vorteil ist weiterhin, wenn der Betätigungstaster zwei Tasterbauteile aufweist, wobei eines dieser Tasterbauteile primär eine technische Funktion erfüllt. Dieses erste Tasterbauteil kann insbesondere beweglich am ersten Gehäusebauteil gelagert sein. Weiterhin kann es einen Verbindungsbereich zur Verbindung mit einer verlagerbaren Pumpkammerwandung aufweisen und/oder einen Krafteinkopplungsbereich zur Einleitung der Kraft einer Rückstellfeder aufweisen.

Das zweite Tasterbauteil ist außenseitig am ersten Tasterbauteil angebracht und weist eine Druckfläche zur unmittelbaren manuellen Kraftbeaufschlagung auf. Dieses zweite Tasterbauteil kann mittels einer Rastverbindung am ersten Tasterbauteil befestigt sein. Das zweite Tasterbauteil ist wie auch das Außengehäusebauteil einfach anpassbar in Hinblick auf seine ästhetische Gestaltung als auch in Hinblick auf die Gestaltung und Anordnung einer Druckfläche zur Kraftbeaufschlagung. Von besonderem Vorteil ist es, wenn das erste Tasterbauteil derart gestaltet ist, dass es bei noch nicht angebrachtem zweiten Tasterteil gestattet, dass das Außengehäusebauteil von der Seite der Abgabeöffnung aus über das erste und das zweite Gehäusebauteil geschoben wird. Das anschließend aufgesetzte zweite Tasterteil kann dann eine Sicherung gegen Abziehen des Außengehäusebauteils darstellen.

Statt eines solchen separaten zweiten Tasterbauteils ist auch eine Bauweise möglich, bei der am Außengehäusebauteil eine Tasterzunge vorgesehen ist, die zum überwiegenden Teil gegenüber dem umgebenden Außengehäusebauteil freigeschnitten ist, jedoch an einer Seite mit diesem einstückig oder gelenkig verbunden ist. Diese Tasterzunge wirkt dann statt des zweiten Tasterbauteils auf das erste Tasterbauteil.

Vorzugsweise weist ein Spender gemäß einem der drei genannten Erfindungsaspekte eine abnehmbare Spenderkappe auf, die auf das Gehäuse des Spenders aufsetzbar ist und von diesem zur Nutzung des Spenders abgenommen wird. Dieser Spenderkappe schützt im aufgesetzten Zustand die Abgabeöffnung. Insbesondere vorzugweise ist eine solche Spenderkappe als belüftete Spenderkappe ausgestaltet und weist hierfür mindestens eine Ventilationsöffnung auf, durch die die Abgabeöffnung auch bei aufgesetzter Spenderkappe mit einer umgebenden Atmosphäre verbunden ist. Dies begünstigt ein schnelles Trocknen eines dort verbliebenen Resttropfens. Im Lieferzustand ist die Ventilationsöffnung vorzugsweise verschlossen und wird vom Benutzer im Zuge der Erstbenutzung geöffnet. Die Spenderkappe verfügt vorzugsweise über einen Sterilfilter, der die Ventilationsöffnungen überdeckt, um Keimeintrag zu verhindern. Weiterhin weist die Spenderkappe vorzugsweise ein integriertes Pad auf, welches bei aufgesetzter Spenderkappe derart über oder an der Abgabeöffnung positioniert ist, dass ein stromabwärts der Abgabeöffnung verbleibender Flüssigkeitsrest hiervon aufgenommen und/oder dekontaminiert wird.

Der Flüssigkeitsspeicher eines erfindungsgemäßen Spenders ist vorzugsweise an dem der Abgabeöffnung gegenüberliegenden Ende des Spenders vorgesehen. Er kann durch einen separaten Flaschenkörper gebildet sein, der an dem die Pumpeinrichtung umgebenden Gehäuse angekoppelt ist, beispielsweise angeschraubt ist. Bei einer hiervon abweichenden Gestaltung ist eine Mantelwandung des Flüssigkeitsspeichers einstückig mit dem Gehäuse verbunden, wobei vorzugsweise ein Boden des Flaschenkörpers durch ein separates Teil gebildet wird, welches an der Mantelwandung befestigt ist. Sofern der Flüssigkeitsspeicher ein unveränderliches Volumen aufweist, verfügt der Spender vorzugsweise über einen Belüftungskanal, insbesondere mit Sterilfilter, durch den der Flüssigkeitsspeicher mit einer umgebenden Atmosphäre verbunden ist. Alternativ dazu kann der Flüssigkeitsspeicher jedoch auch ein variables Innenvolumen aufweisen, insbesondere indem ein Beutel vorgesehen ist, in dem die Flüssigkeit gelagert ist und der von einem starren Flaschenkörper umgeben ist. Auch ein Schleppkolbensystem gestattet die Realisierung eines variablen Innenvolumens.

Ein erfindungsgemäßer Spender ist zur Verwendung für den Austrag pharmazeutischer Flüssigkeiten vorgesehen. Daher ist in einem Lieferzustand der Flüssigkeitsspeicher vorzugweise mit einer solchen pharmazeutischen Flüssigkeit befüllt. Insbesondere handelt es sich um pharmazeutische Flüssigkeiten zur Behandlung des erhöhten Augeninnendrucks (Glaukombehandlung), zur Behandlung des trockenen Auges und zur Behandlung von Allergien und Entzündungen. Hierbei spielen insbesondere die Molekülgruppen Alpha-2 Agonisten, z. B. Brimonidin, Prostaglandinanaloga (Tafluprost, Latanoprost, Bimatoprost, Travoprost), Beta-Blocker, z. B. Timolol, und Carboanhydrasehemmer, z. B. Dorzolamid beziehungsweise Hyaluronsäureverbindungen, Filmbildner, z. B. Methylcelluloseverbindungen und Cyclosporin beziehungsweise Antihistaminika, z. B. Olopatadin und Levocabastin, Steroide, z. B. Loteprdnol und Dexamethason, sowie NSAID, z. B. Keterolac, eine Rolle.

Weiterhin ist der erfindungsgemäße Spender vorteilhaft verwendbar für Flüssigkeiten mit Molekülen von einer oder von mehreren der folgenden Sorten : Trichloressigsäure, Trioxysalen, Harnstoff, Zinkoxid, Tacrolimus, Clobetasolpropionat, Mometasonfuroat, Betamethasondipropionat, Fluocinonid, Desoximetasone, Triamcinolon Acetonid, Fluticasonpropionat, Hydrokortison, Clotrimazol, Ketoconazol, Miconazol, Undecylensäure, Terbinafine, Cyclopirox, Tolnaftate, Akziklovir, Imiquimod, Docosanol, Finasterid, Minoxidil, Dexamethason, Tramazolin, Naphazolin, Nostrilla, Oxymethazolin, Phenylephrin, Phenylpropanolamin, Pseudoephedrin, Tetryzolin, Tramazolinhydrochlorid, Tuaminoheptane und Xylometazolin.

Die Komponenten eines erfindungsgemäßen Spenders sind vorzugsweise aus Kunststoff gefertigt. Lediglich für die beschriebenen Federn und ggf. vorhandene Federn am Einlassventil und/oder dem Auslassventil stellt eine metallische Ausgestaltung eine Alternative dar. Die im Betrieb bestimmungsgemäß verformbaren Teile, insbesondere also der überwiegende Teil des Membranbauteils, die Ventillippen und der Abgabeventilkörper bestehen vorzugsweise aus einem Elastomer-Kunststoff mit einem E-Modul < 200 N/mm². Es kommt beispielsweise ein LDPE (Low Density Polyethylen) in Frage. Die im Betrieb unbeweglichen Teile, insbesondere alle oder einige Gehäuseteile,bestehen vorzugsweise aus einem Kunststoff mit einem E-Modul > 500 N/mm². Hierbei kann es sich beispielsweise um ein HDPE (High Density Polyethylen) oder ein PP (Polypropylen) handeln.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1A und 1B zeigen einen erfindungsgemäßen Spender, umfassend eine Haupteinheit sowie Spenderkappe in einer ungeschnittenen Darstellung.
Fig. 2A und 2B zeigen den Spender in einer geschnittenen Darstellung mitsamt der innenliegenden Pumpkammer, die durch ein Membranteil und ein Ventilbauteil begrenzt wird.
Fig. 3 und 4 zeigen den Spender in perspektivischen geschnittenen Darstellungen.
Fig. 5 und 6 zeigen den Betätigungsvorgang des Spenders.
Anhand von Fig. 7 wird die Montagereihen der Einzelteile des Spenders verdeutlicht.
Fig. 8 zeigt die Kopplung zwischen einem dem Betätigungstaster des Spenders und dem Membranteil.
Fig. 9 und 10 zeigen in Details das Ventilbauteil sowie das Membranteil.
Fig. 11 zeigt eine alternative Bauweise des Spenders.

### DETAILLIERTE BESCHREIBUNG DER AUSFÜHRUNGSBEISPIELE

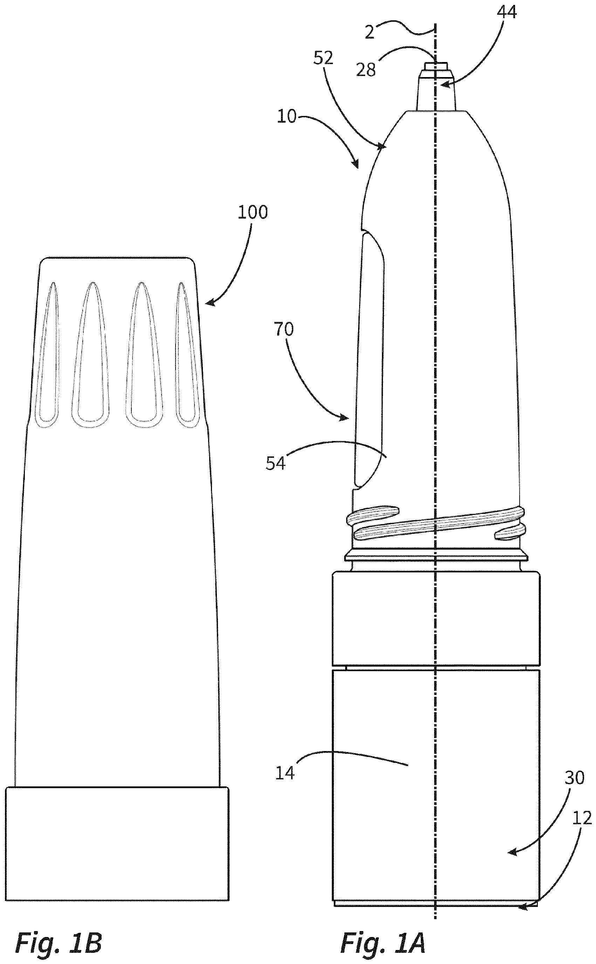

Die Fig. 1A und 1B zeigen ein erstes Ausführungsbeispiel des erfindungsgemäßen Spenders in einer nicht geschnittenen Darstellung mitsamt seiner Spenderkappe. Die in Fig. 1A dargestellte Haupteinheit dieser Ausgestaltung des Spenders weist ein längliches Gehäuse auf, welches aus verschiedenen Gehäusebestandteilen 30, 12, 52, 44 zusammengesetzt ist, die entlang einer Haupterstreckungsrichtung 2 ausgerichtet sind. Wie im Weiteren noch beschrieben ist, ist innerhalb des Gehäuses eine Pumpeinrichtung 16 vorgesehen, mittels der Flüssigkeit aus einem Flüssigkeitsspeicher 14 an einem proximalen Ende des Spenders 10 zu einer Abgabeöffnung 28 an einem distalen Ende des Spenders 10 gefördert werden kann. Zur Betätigung der Pumpeinrichtung 16 ist ein Betätigungstaster 70 vorgesehen.

Die in Fig. 1B dargestellte Spenderkappe 100 kann mittels eines Gewindes auf das Gehäuse des Spenders 10 aufgesetzt werden.

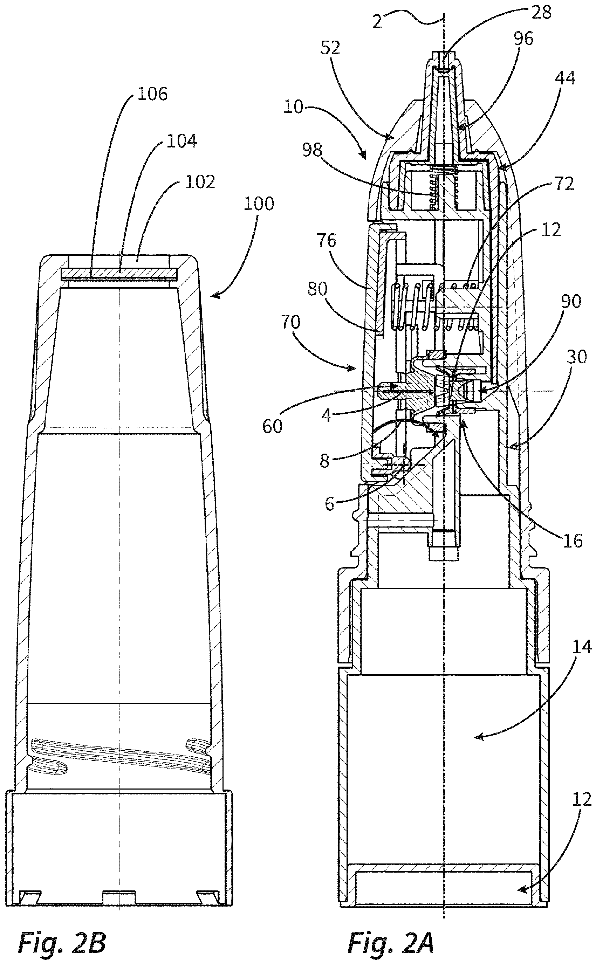

Die Fig. 2A und 2B zeigen die Haupteinheit des Spenders 10 sowie die Spenderkappe 100 in geschnittener Darstellung.

Anhand dieser Darstellung sollen zunächst die Hauptelemente beschrieben werden.

Wie in Fig. 2A ersichtlich ist, besteht die Haupteinheit des Spenders 10 aus mehreren Gehäuseteilen, von denen das erste Gehäusebauteil 30 das größte Gehäusebauteil ist und gleichsam einen zentralen Träger für weitere Bauteile des Spenders darstellt. Dieses Gehäusebauteil 30 stellt an seinem proximalen Ende die Mantelwandung des Flüssigkeitsspeichers 14 zur Verfügung. Es ist Träger verschiedener Bestandteile der Pumpeinrichtung, insbesondere eines Membranteils 60 und eines Ventilbauteils 90, die auf gegenüberliegenden Seiten einer Pumpkammer 20 angeordnet sind. Der Betätigungstaster 70 ist um eine Kippachse 6 schwenkbeweglich am ersten Gehäusebauteil 30 angelenkt. Der Betätigungstaster 70 selbst besteht aus zwei Tasterbauteilen 76, 80, wobei das innenliegende Tasterbauteil 80 in im Weiteren noch beschriebener Art und Weise mit dem Membranteil 60 verbunden ist, so dass das Membranteil 60 mittels des Betätigungstasters 70 in die Pumpkammer 20 eindrückbar ist. Zwischen dem Tasterbauteil 80 und dem ersten Gehäusebauteil ist eine Rückstellfeder 72 vorgesehen.

Am distalen Ende des ersten Gehäusebauteils 30 schließt sich ein zweites Gehäusebauteil 44 an. Dieses zweite Gehäusebauteil 44 ist ortsfest am ersten Gehäusebauteil 30 vorgesehen und dichtet mit diesem gemeinsam einen Innenraum ab, innerhalb dessen ein verformbarer Abgabeventilkörper 96 angeordnet ist, dem seinerseits eine Abgabeventilfeder 98 zugeordnet ist. Ein weiteres Gehäusebauteil, das Außengehäusebauteil 52, umgibt die genannten Gehäusebauteile 30, 44, wobei das Gehäusebauteil 44 mit einer Applikatorspitze 46 durch eine Durchbrechung 58 des Außengehäusebauteils 52 hindurchragt und wobei das Außengehäusebauteil 52 eine Durchbrechung 56 in einer Mantelfläche 54 aufweist, durch die hindurch der Betätigungstaster 70 in der durch den Pfeil 8 verdeutlichten Art und Weise eingedrückt werden kann, um hierdurch das Membranteil 60 in der durch den Pfeil 4 verdeutlichten Richtung zu verlagern.

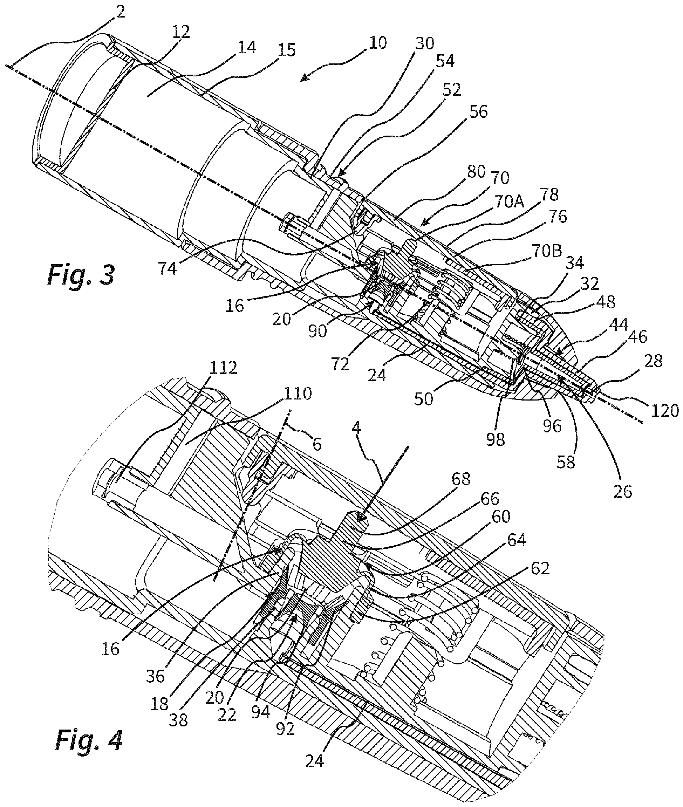

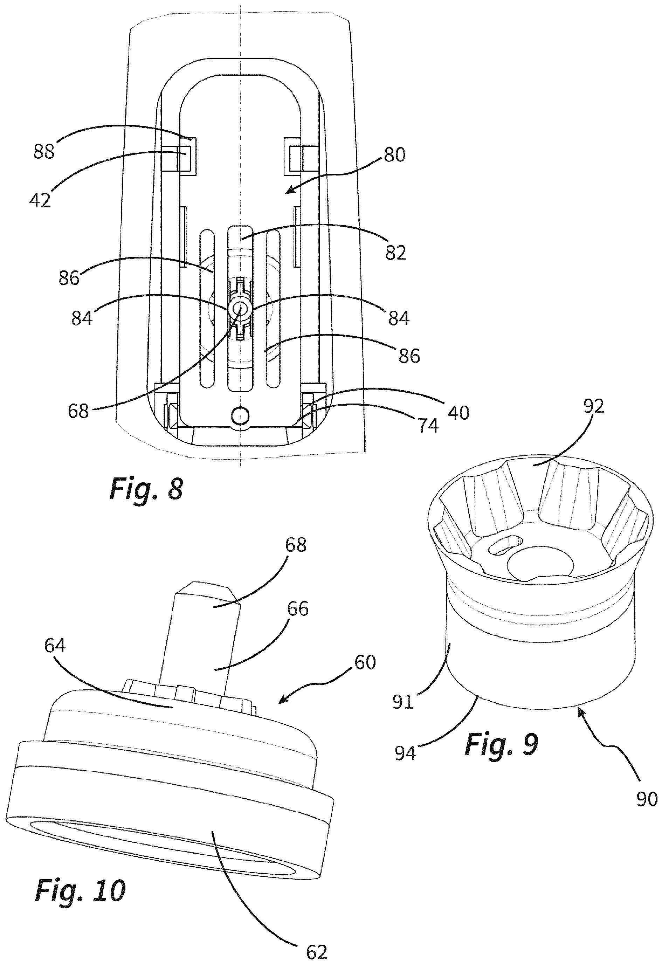

Anhand der perspektivischen Darstellungen der Fig. 3 und 4 sowie Bezug nehmend auf die Detaildarstellungen der Fig. 8 bis 10 wird der Spender weiter erläutert.

Vor dem Flüssigkeitsaustrag ist die Flüssigkeit im Flüssigkeitsspeicher 14 gelagert, welcher im Falle des Ausführungsbeispiels der Fig. 1A bis 10 durch eine Mantelfläche 15 begrenzt wird, die Teil des ersten Gehäusebauteils 30 ist, und der am proximalen Ende des Spenders durch einen eingerasteten Boden 12 verschlossen wird. Der Spender 10 wird üblicherweise in einer Ausrichtung verwendet, in der die Abgabeöffnung 28 nach unten weist, so dass die Flüssigkeit im Flüssigkeitsspeicher 14 während der Betätigung des Betätigungstasters 70 unmittelbar am Ventilbauteil 90 anliegt, nämlich an einer Einlassventillippe 92 des Einlassventils 18. Bei Unterdruck in der sich daran anschließenden Pumpkammer 20 kann die Flüssigkeit somit in die Pumpkammer 20 einströmen. Die Pumpkammer 20 selbst ist durch das genannte Ventilbauteil 90, einen ringförmigen Gehäuseabschnitt 36 des ersten Gehäusebauteils 30 sowie das bereits genannte in sich verformbare Membranteil 60 begrenzt.

Wenn das Membranteil in Richtung des Pfeils 4 und in Richtung des Ventilbauteils 90 verlagert wird, stellt sich ein Überdruck in der Pumpkammer ein, der ein Auslassventil 22 öffnet, indem dessen Auslassventillippen 94 ausgelenkt werden. Die Flüssigkeit kann hierdurch in einen Kanalabschnitt 24 strömen, der in Haupterstreckungsrichtung 2 ausgerichtet ist und bis zum distalen Ende des Gehäusebauteils 30 führt. An diesem distalen Ende des Gehäusebauteils 30 sind zwei ringförmige Stege 32, 34 vorgesehen. Der äußere Steg 32 weist einen Innendurchmesser auf, der an einen ringförmigen Steg 48 des zweiten Gehäusebauteils 44 derart angepasst ist, dass ein durch die beiden Gehäusebauteile 30, 44 definierter Zwischenraum gegenüber der Umgebung isoliert ist. An dem ringförmigen Steg 48 des zweiten Gehäusebauteils 44 ist ein lanzenförmiger Fortsatz vorgesehen, der einen Einsatz 50 bildet, welcher in den Kanalabschnitt 24 hineinragt und dessen Volumen zum überwiegenden Teil ausfüllt. Die Flüssigkeit, die durch den so bezüglich seines Volumens minimierten Kanalabschnitt 24 bis zum zweiten Gehäusebauteil 44 geströmt ist, kann hier nicht unmittelbar durch die Abgabeöffnung 28 austreten, da ein Abgabeventil 26 der Abgabeöffnung 28 vorgeschaltet ist, welches den bereits genannten Abgabeventilkörper 96 umfasst, der mittels der Abgabeventilfeder 98 abdichtend gegen die Abgabeöffnung 28 gedrückt wird und diese somit verschließt. Mit steigendem Flüssigkeitsdruck am Abgabeventil 26 wird jedoch der Abgabeventilkörper 96 gegen die Kraft der Abgabeventilfeder 98 verformt und öffnet somit den Flüssigkeitspfad, so dass die Flüssigkeit unter vergleichsweise geringem Druck durch die Abgabeöffnung 28 austreten kann und an den jenseits der Abgabeöffnung 28 vorgesehenen Tropfenbildungsmitteln in Form einer Tropfenbildungsfläche 120 einen Tropfen bildet. Erst wenn dieser ein durch die Geometrie derTropfenbildungsmittel definiertes Volumen aufweist, löst er sich und kann somit beispielsweise in ein Auge, ein Nasenloch oder ein Ohr des Benutzers appliziert werden.

Wenn der Betätigungstaster 70 nach erfolgter Betätigung vom Benutzer losgelassen wird, wird er von der in Art einer metallischen Schraubenfeder ausgestalteten Federeinrichtung 72 zurück in seine Ausgangslage gedrückt. Hierbei wird das Membranteit 60 mitgezogen und das Volumen der Pumpkammer 20 wieder vergrößert, so dass Flüssigkeit aus dem Flüssigkeitsspeicher 14 in die Pumpkammer 20 eingesogen wird. Um zu verhindern, dass hierdurch ein Unterdruck im Flüssigkeitsspeicher 14 entsteht, weist der Spender 10 einen Belüftungskanal 110 auf, der mit einem Sterilfilter 112 versehen ist und der ein Nachströmen von Luft aus einer Umgebung in den Flüssigkeitsspeicher 14 gestattet.

Sowohl das genannte Ventilbauteil 90 als auch das Membranteil 60 sind jeweils als Bauteil ausgestaltet, welches im Zweikomponentenspritzguss hergestellt wird. Beide Bauteile weisen jeweils einen starren Bauteilabschnitt auf, nämlich einen Befestigungsring 91, 62, an dem jeweils eine Komponente aus weichem Kunststoff angespritzt ist. Im Falle des in Fig. 9 vergrößert dargestellten Ventilbauteils bildet der weiche Kunststoff insbesondere die bereits genannten Komponenten der Einlassventillippe 92 und der aufgrund der Perspektive in Fig. 9 nicht erkennbaren Auslassventillippe 94. Im Falle des Membranteils 60 bilden die aus weichem Kunststoff hergestellten Bestandteile eine Pumpkammerwandung 64, die in Richtung der Pumpkammer 20 weist, und ein dickeres Mittelteil, welches auf der der Pumpkammer abgewandten Seite in einen Ausgleichsabschnitt 66 und einen Verbindungsabschnitt 68 übergeht.

Bezug nehmend wieder auf die geschnittenen Darstellungen der Fig. 4, in der das äußere Tasterbauteil 76 ausgeblendet ist, ist zu erkennen, dass der genannte Verbindungsabschnitt 68 in einem Verbindungsbereich 70A in das Tasterbauteil 80 hineinragt. Er ist hier mit einer Klemmverbindung gesichert. Diese Klemmverbindung ist anhand der Fig. 8 näher erläutert, welche einen Ausschnitt des Spenders bei abgenommenem Tasterbauteil 76 zeigt. Wie hier zu erkennen ist, sind in dem Tasterbauteil 80 drei Durchbrechungen 82, 86 vorgesehen. Die Durchbrechung 82 ist die eigentliche Kopplungsdurchbrechung. Sie ist in Form einer länglichen Durchbrechung gestaltet, deren einander gegenüberliegenden Längsseiten Klemmflächen 84 zur Festlegung des Verbindungsabschnitts bilden. Diese sind mit einer scharfkantigen Profilierung versehen, die sich von beiden Seiten in den Verbindungsabschnitt 68 hineindrücken und somit die gewünschte feste Klemmverbindung schaffen. Die hierzu parallel ausgerichteten Durchbrechungen 86 bilden Montagedurchbrechungen, die dem Zweck dienen, während der Montage eine Auslenkung der Stege zwischen der Kopplungsdurchbrechung und den Montagedurchbrechungen zu ermöglichen, ohne dass diese Stege oder andere Teile des Tasterbauteils 80 beschädigt werden.

Ebenfalls Bezug nehmend auf Fig. 8 wird die Befestigung des Tasterbauteils 80 erläutert. Das Tasterbauteil 80 verfügt über zwei angeformte Achsabschnitte 74, die die Kippachse 6 definieren. Diese Achsabschnitte 74 sind seitlich in Lagerösen 40 gelagert, die Teil des ersten Gehäusebauteils 30 sind.

Am gegenüberliegenden Ende des Betätigungstasters 70 verfügt dieser über zwei ähnlich ausgestaltete Führungsösen 88, in die Nocken 42 des ersten Gehäusebauteils 30 hineinragen. Hierdurch wird verhindert, dass die Federeinrichtung 72 das Tasterbauteil 80 sowie den Kipptaster 70 als Ganzes aus dem Gehäuse herausdrückt.

Wie anhand der Fig. 3 und 4 gut zu ersehen ist, sind ein Verbindungsbereich 70A, in dem das Tasterbauteil 80 mit dem Verbindungsabschnitt 68 des Membranteils 60 verbunden ist, und ein Krafteinkopplungsbereich 70B, in dem die Federeinrichtung 72 auf das Tasterbauteil 76 wirkt, unterschiedlich weit von der Kippachse 6 beabstandet. Dies erleichtert zum einen die Montage der Einzelteile im begrenzten Bauraum. Zum anderen kann hierdurch die Auslegung des Spenders und die auf den Betätigungstaster 70 wirkende Rückstellkraft in vorteilhafter Weise angepasst werden.

Wie sich aus Fig. 8 ersehen lässt, ist das dort dargestellte innere Tasterbauteil 80 bezüglich seiner Formgebung vor allem im Hinblick auf seine technische Funktion ausgestaltet. Um dennoch eine ästhetisch ansprechende Gestaltung des Betätigungstasters 70 zu erzielen, ist das zweite, äußere Tasterbauteil 76 vorgesehen, welches mittels einer Rastverbindung mit dem inneren Tasterbauteil 80 verbunden ist. An diesem äußeren Tasterbauteil 76 ist die Druckfläche 78 vorgesehen, auf die ein Benutzer zum Zwecke der Betätigung seinen Daumen legt.

Wie bereits erläutert wurde, ist der dargestellte Spender als Tropfenspender ausgebildet. Dabei ist er derart ausgelegt, dass eine Betätigung des Betätigungstasters von einer unbetätigten Ausgangslage bis in seine betätigte Endlage den Austrag von genau einem Tropfen bewirken soll. Dementsprechend ist die Pumpkammer 20 recht klein ausgebildet und weist ein nutzbares Pumpkammervolumen, also eine Differenz zwischen Maximal- und Minimalvolumen der Pumpkammer 20, von 40 µl auf.

Dieses sehr kleine Pumpvolumen führt dazu, dass besondere Vorkehrungen getroffen werden müssen, um den Spender in Betrieb zu nehmen. Im Lieferzustand des Spenders ist die Pumpkammer 20 mit Luft gefüllt. Ebenso ist der Flüssigkeitspfad von der Pumpkammer 20 bis zur Abgabeöffnung 28 mit Luft gefüllt.

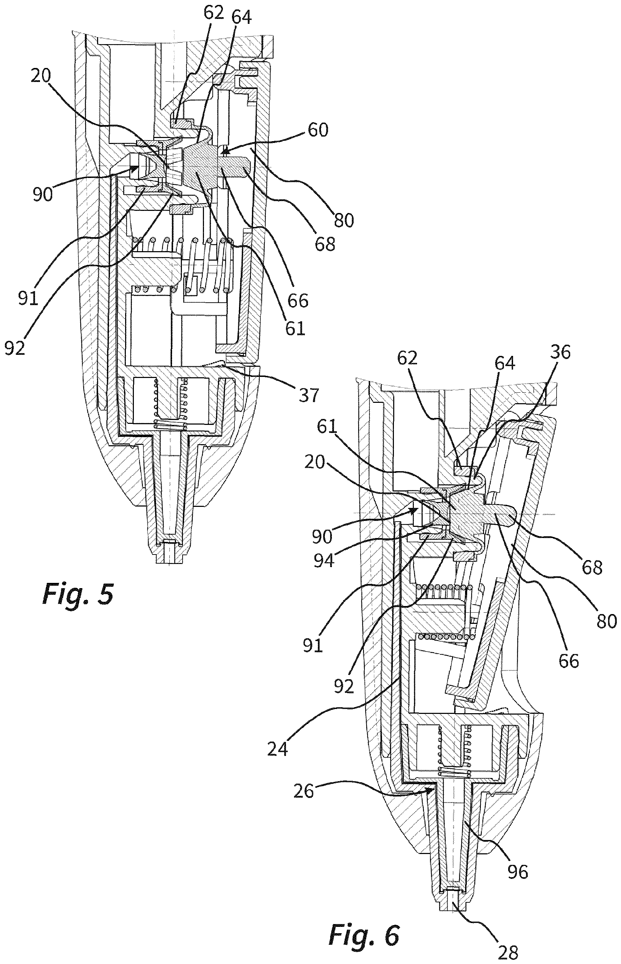

Damit es ausgehend von diesem Ausgangszustand überhaupt möglich ist, die Luft aus der Pumpkammer 20 zu verdrängen, weist die Pumpeinrichtung 16 einen sehr kleinen Quotienten zwischen dem Minimalvolumen der Pumpkammer 20 im betätigten Zustand und dem Maximalvolumen der Pumpkammer 20 im unbetätigten Zustand auf. Dies wird anhand der Fig. 5 und 6 dargestellt. Fig. 5 zeigt den unbetätigten Zustand. Fig. 6 zeigt den betätigten Zustand.

Es ist zu ersehen, dass im betätigten Zustand das Membranteil 60 derart weit in Richtung des auf einer ringförmigen Gegenwandung 38 aufgesetzten Ventilbauteils 90 verlagert ist, dass es mit diesem in Berührkontakt gelangt. Dort, wo das Membranteil 60 und das Ventilbauteil 90 in Berührkontakt gelangen, verbleibt kein Restvolumen zwischen ihnen. Es ist weiter zu ersehen, dass die Pumpkammerwandung 64, die durch das Membranteil 60 gebildet wird, sich bei der Überführung in den betätigten Zustand partiell umstülpt bzw. abrollt, so dass sie gegen Ende der Betätigung am ringförmigen Gehäuseabschnitt 36 anliegt oder nur noch ein sehr schmaler Spalt hierzwischen verbleibt. Das hierdurch erzielbare Minimalvolumen der Pumpkammer, welches in Fig. 6 dargestellt ist, beträgt weniger als 2 µl. Der Quotient aus Minimalvolumen und Maximalvolumen der Pumpkammer 20 liegt somit unter 1:20.

Zu erkennen ist anhand der Fig. 5 und 6 auch, dass die Kippbeweglichkeit des Betätigungstasters dazu führt, dass auch der Verbindungsabschnitt 68 des Membranteils 60 entsprechend verkippt wird. Um dennoch das genannte sehr geringe Minimalvolumen der Pumpkammer 20 zu ermöglichen, verformt sich der Ausgleichsabschnitt 66 in der in Fig. 6 dargestellten Weise.

Eine ähnliche Problematik wie bei der Pumpkammer 20 ist auch im Hinblick auf den Flüssigkeitspfad bis zur Abgabeöffnung 28 vorhanden. Auch hier ist es von Vorteil, wenn das Volumen möglichst gering ist, damit hier im Lieferzustand nur wenig Luft vorhanden ist, die vor dem Austrag ausgetrieben werden muss.

Hier ist es insbesondere die schon beschriebene Gestaltung des Einsatzes 50, die das Volumen stark reduziert, so dass die aus der Pumpkammer 20 in Richtung des Kanalabschnitts 24 ausgetriebene Luft nach zwei bis drei Betätigungsvorgängen des Betätigungstasters 70 einen ausreichenden Druck erreicht hat, um das Abgabeventil 26 zu öffnen.

In den Fig. 5 und 6 ist gestrichelt auch eine optionale Raste 37 vorgesehen, die bei Betätigung eine Mindestkraft erzwingt. Dies ist insbesondere zweckmäßig, wenn der Spender für ein Pumpvolumen ausgebildet ist, welches geringer als jenes ist, das für eine rein schwerkraftbedingt Ablösung des Tropfens erforderlich ist. Bei derart geringen Volumina kann der durch die schlagartige Pumpkammerkomprimierung erzeugte Druckstoß dennoch ein Ablösen des Tropfens von der Tropfenbildungsfläche 120 bewirken.

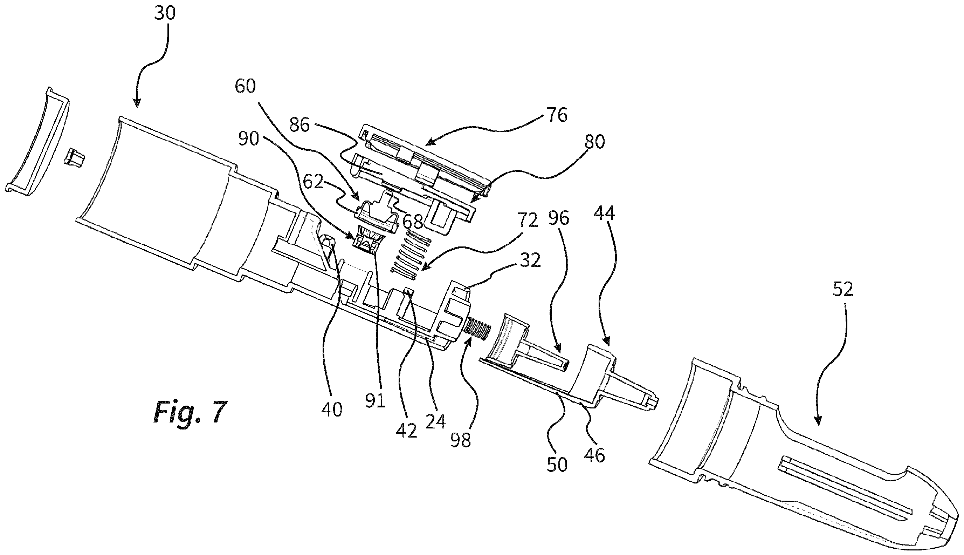

Anhand der Fig. 7 wird nachfolgend die Montagereihenfolge erläutert.

Ausgehend von dem ersten Gehäusebauteil 30 wird zunächst das Ventilbauteil 90 eingefügt und mittels seines Befestigungsrings 91 an der ringförmigen Gegenwandung 38 des ersten Gehäusebauteils 30 befestigt. Anschließend werden die Rückstellfeder 72 und das Membranteil 60 eingefügt, wobei das Membranteil 60 mittels eines Befestigungsrings 62 an der Außenseite des ringförmigen Gehäuseabschnitts 36 festgeklemmt wird und hierbei eine flüssigkeitsdichte Verbindung schafft.

Anschließend erfolgt die Einfügung des inneren Tasterbauteils 80, welches hierbei im Bereich der Achsabschnitte 74 in die Lagerösen 40 hineingedrückt wird und im Bereich der Führungsösen 88 über die am ersten Gehäusebauteil 30 vorgesehenen Nocken 42 gedrückt wird. Während der Einfügung des Tasterbauteils 80 wird die Kopplungsdurchbrechung 82 von einem Spreizer geweitet, so dass der Verbindungsabschnitt 68 des Membranteils ohne Verformung in die Kopplungsdurchbrechung einfahren kann. Anschließend wird der Spreizer entfernt und die beschriebene Klemmverbindung so geschaffen.

Danach wird ein vormontierter Verbund von verformbarem Abgabeventilkörper 96 und zweitem Gehäusebauteil 44 stirnseitig auf das erste Gehäusebauteil 30 aufgeschoben, wobei zuvor die Abgabeventilfeder 98 an dieser Stirnseite aufgesetzt wurde und wobei weiterhin der Einsatz 50 in den Kanalabschnitt 24 des ersten Gehäusebauteils 30 eingefügt wird.

Als letzter Fertigungsschritt wird zunächst das Außengehäusebauteil 52 auf den Verbund der zuvor montierten Teile aufgeschoben, wobei hierdurch auch eine Sicherung des ersten Gehäusebauteils 30 und des zweiten Gehäusebauteils 44 aneinander erzielt wird. Anschließend wird das zweite Tasterbauteil 76 auf das erste Tasterbauteil 80 gedrückt und verrastet hier.

Die beiden letztgenannten Bauteile 52, 76 haben keinerlei Flüssigkeitskontakt und beeinflussen die Austragcharakteristik des Spenders daher nicht. Diese beiden Bauteile 52, 76 sind jene Bauteile, die bestimmungsgemäß im Hinblick auf Form und Farbe angepasst werden können, um den Spender 10 an individuelle Wünsche eines Herstellers von pharmazeutischen Flüssigkeiten anpassen zu können.

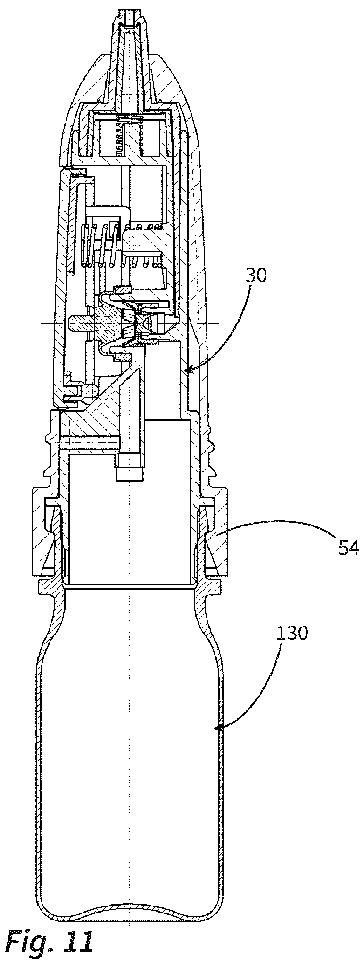

Fig. 11 zeigt ein zweites Ausführungsbeispiel eines erfindungsgemäßen Spenders. Dieser weist nur einen einzigen Unterschied zum vorgenannten Spender auf, nämlich eine Ausgestaltung des ersten Gehäusebauteils 30 und des Außengehäusebauteils 52 derart, dass diese die Ankopplung eines separaten Flaschenkörpers 130 gestatten.

## Patentansprüche

1. Spender (10) zum Austrag pharmazeutischer Flüssigkeiten, insbesondere für den tropfenweisen Austrag von pharmazeutischen Flüssigkeiten in Form von Einzeltropfen, mit den folgenden Merkmalen:
a. der Spender (10) weist einen Flüssigkeitsspeicher (14), eine Pumpeinrichtung (16) mit einer volumetrisch veränderlichen Pumpkammer (20) sowie eine Abgabeöffnung (28) zur Abgabe der Flüssigkeit auf, und
b. der Spender (10) weist ein entlang einer Haupterstreckungsrichtung (2) ausgerichtetes längliches Gehäuse auf, wobei an einem distalen Ende des Spenders (10) die Abgabeöffnung (28) vorgesehen ist, und
c. der Spender (10) weist einen Betätigungstaster (70) auf, der im Bereich einer Mantelfläche (54) des Gehäuses seitlich am Spender (10) vorgesehen ist und der zwischen einer unbetätigten Ausgangslage und einer betätigten Endlage eindrückbar ist, und
d. die volumetrisch veränderliche Pumpkammer (20) ist an einer Seite durch eine verlagerbare und/oder verformbare Pumpkammerwandung (64) begrenzt, die mit dem Betätigungstaster (70) in einem Verbindungsbereich (70A) derart verbunden ist, dass sie durch Eindrücken des Betätigungstasters (70) die Pumpkammer verkleinert, und
e. der Spender (10) weist eine Federeinrichtung (72) auf, die in einem Krafteinkopplungsbereich (70B) auf den Betätigungstaster (70) wirkt und durch die der Betätigungstaster (70) in Richtung seiner unbetätigten Ausgangslage kraftbeaufschlagt ist, und
f. der Betätigungstaster (70) ist als Kipptaster (70) ausgebildet, der um eine Kippachse (6) kippbeweglich gegenüber dem Gehäuse ausgebildet ist,
**gekennzeichnet durch** das folgende Merkmal:
g. der Krafteinkopplungsbereich (70B) und der Verbindungsbereich (70A) sind unterschiedlich weit von der Kippachse (6) entfernt am Kipptaster (70) vorgesehen.

2. Spender (10) nach Anspruch 1 mit dem folgenden weiteren Merkmal:
a. die Kippachse (6) ist bezogen auf die Haupterstreckungsrichtung (2) an einem von der Abgabeöffnung (28) weg weisenden Ende des Kipptasters (70) vorgesehen.

3. Spender (10) nach Anspruch 1 oder 2 mit dem folgenden weiteren Merkmal:
a. auf der der Kippachse (6) abgewandten Seite des Kipptasters (70) ist eine Raste (37) vorgesehen, die ein Eindrücken des Kipptasters (70) erschwert und vorzugsweise zur Überwindung eine Betätigungskraft von mindestens 10 Newton an dem der Kippachse (6) abgewandten Ende des Kipptasters (70) erfordert.

4. Spender (10) nach einem der vorstehenden Ansprüche mit mindestens einem der folgenden zusätzlichen Merkmale:
b. der Krafteinkopplungsbereich (70B) ist weiter von der Kippachse (6) entfernt als der Verbindungsbereich (70A) und/oder
c. der Abstand zwischen der Kippachse (6) und dem Krafteinkopplungsbereich (70B) und der Abstand zwischen der Kippachse (6) und dem Verbindungsbereich (70A) weichen voneinander um mindestens 20% bezogen auf den kleineren der Abstände ab.

5. Spender (10) nach einem der vorstehenden Ansprüche mit mindestens einem der folgenden weiteren Merkmale:
a. zwischen dem Kipptaster (70) und der verlagerbaren und/oder verformbaren Pumpkammerwandung (64) ist ein elastisch verformbarer Ausgleichsabschnitt (66) vorgesehen, und/oder
b. die Pumpkammerwandung (64) wird durch ein verformbares Membranteil (60) gebildet.

6. Spender (10) nach einem der vorstehenden Ansprüche mit dem folgenden weiteren Merkmal:
a. am Kipptaster (70) ist mindestens ein Achsabschnitt (74) vorgesehen, der in einem gehäuseseitigen Lager (40) aufgenommen ist.

7. Spender (10) nach einem der vorstehenden Ansprüche mit den folgenden weiteren Merkmalen:
a. der Spender (10) weist einen in Haupterstreckungsrichtung (2) ausgerichteten Kanalabschnitt (24) auf, der stromabwärts der Pumpkammer (20) vorgesehen ist und durch den Flüssigkeit zur Abgabeöffnung (28) förderbar ist, und
b. der Spender (10) weist einen Einsatz (50) auf, der von einem distalen Ende aus in den Kanalabschnitt (24) eingeschoben ist, um dessen freies Volumen zu reduzieren.

8. Spender (10) nach Anspruch 7 mit den folgenden weiteren Merkmalen:
a. der Spender (10) weist ein erstes Gehäusebauteil (30) auf, in dem der Kanalabschnitt (24) vorgesehen ist, und
b. der Spender (10) weist ein zweites Gehäusebauteil (44) auf, das am distalen Ende des ersten Gehäusebauteils (30) angeordnet ist und das von der Abgabeöffnung (28) durchdrungen ist, und
c. der Einsatz (50) ist am zweiten Gehäusebauteil (44) vorgesehen.

9. Spender (10) nach Anspruch 8 mit den folgenden weiteren Merkmalen:
a. das erste Gehäusebauteil (30) weist an seinem distalen Ende einen ersten umlaufenden Steg (32) auf, und
b. das zweite Gehäusebauteil (44) weist einen zweiten umlaufenden Steg (48) auf, und
c. eine Dichtfläche an der Außenseite des zweiten umlaufenden Stegs (48) liegt unter Bildung eines umlaufenden Dichtbereichs an einer Innenseite des ersten umlaufenden Stegs (32) an,
insbesondere mit dem folgenden zusätzlichen Merkmal:
d. der Einsatz (50) ist als Fortsatz am zweiten umlaufenden Steg (48) vorgesehen.

10. Spender (10) nach einem der vorstehenden Ansprüche mit den folgenden weiteren Merkmalen:
a. das Gehäuse weist ein erstes Gehäusebauteil (30) auf, welches die Pumpkammer (20) zumindest abschnittsweise begrenzt, und
b. das Gehäuse weist ein zweites Gehäusebauteil (44) auf, welches von der Abgabeöffnung (28) durchdrungen ist, und
c. das Gehäuse weist ein Außengehäusebauteil (52) auf, welches das erste und zweite Gehäusebauteil (30, 44) zumindest teilweise umgibt, wobei das zweite Gehäusebauteil (44) durch eine Durchbrechung (58) des Außengehäusebauteils (52) aus diesem herausragt und wobei eine Durchbrechung (56) zur Betätigung des Betätigungstasters (70) vorgesehen ist, und
d. das Außengehäusebauteil (52) weist keinerlei flüssigkeitsführende Flächen auf, die einen Flüssigkeitspfad zwischen Flüssigkeitsspeicher (14) und Abgabeöffnung (28) begrenzen.

11. Spender (10) nach Anspruch 10 mit dem folgenden weiteren Merkmal:
a. das zweite Gehäusebauteil (44) ist zwischen einem distalen Ende des Außengehäusebauteils (52) und dem distalen Ende des ersten Gehäusebauteils (30) gesichert, vorzugsweise eingeklemmt.

12. Spender (10) nach Anspruch 10 oder 11 mit den folgenden weiteren Merkmalen:
a. der Betätigungstaster (70) weist ein erstes Tasterbauteil (80) auf,
- welches beweglich am ersten Gehäusebauteil (30) gelagert ist, und/oder
- welches einen Verbindungsbereich (70A) zur Verbindung mit einer verlagerbaren Pumpkammerwandung (64) aufweist und/oder
- welches einen Krafteinkopplungsbereich (70B) zur Einleitung der Kraft der Federeinrichtung (72) aufweist, und
b. der Betätigungstaster (70) weist ein zweites Tasterbauteil (76) auf, welches außenseitig am ersten Tasterbauteil (80) angebracht ist und eine Druckfläche (78) zur unmittelbaren manuellen Kraftbeaufschlagung aufweist.

13. Spender (10) nach einem der vorstehenden Ansprüche mit dem folgenden weiteren Merkmal:
a. der Spender (10) ist als Tropfenspender (10) ausgebildet und weist stromabwärts der Abgabeöffnung (28) Tropfenbildungsmittel auf,
vorzugsweise mit mindestens einem der folgenden zusätzlichen Merkmale:
b. die Tropfenbildungsmittel umfassen eine die Abgabeöffnung umgebende Tropfenbildungsfläche, welche vorzugsweise eben oder konkav geformt ist und/oder welche vorzugsweise von einer scharfkantigen Abrisskante umgeben sind, und/oder
c. der Spender weist eine Abstimmung zwischen der Pumpeinrichtung (16) und den Tropfenbildungsmitteln auf, durch die eine vollständige Betätigung des Betätigungstasters genau einen abfallenden Tropfen erzeugt.

14. Spender (10) nach einem der vorstehenden Ansprüche mit dem folgenden weiteren Merkmal:
a. der Spender (10) weist eine Spenderkappe (100) auf, die auf das Gehäuse aufsetzbar ist und im aufgesetzten Zustand die Abgabeöffnung schützt,
vorzugweise mit mindestens einem der folgenden zusätzlichen Merkmale:
b. die Spenderkappe (100) ist als belüftete Kappe ausgestaltet und weist eine Ventilationsöffnung (102) auf, durch die die Abgabeöffnung (28) auch bei aufgesetzter Spenderkappe (100) mit einer umgebenden Atmosphäre verbunden oder verbindbar ist, wobei der Ventilationsöffnung (102) vorzugsweise ein Sterilfilter (104) zugeordnet ist, und/oder
c. die Spenderkappe (100) weist ein Pad (106) auf, welches bei aufgesetzter Spenderkappe (100) derart über oder an der Abgabeöffnung (28) positioniert ist, dass ein stromabwärts der Abgabeöffnung (28) verbleibender Flüssigkeitsrest hiervon aufgenommen und/oder dekontaminiert wird.

15. Spender (10) nach einem der vorstehenden Ansprüche mit mindestens einem der folgenden Merkmale:
a. die Differenz zwischen einem maximalen Pumpkammervolumen und einem minimalen Pumpkammervolumen beträgt zwischen 20 µl und 50 µl, vorzugsweise zwischen 30 µl und 40 µl, und/oder
b. der Quotient zwischen einem minimalen Pumpkammervolumen und einem maximalen Pumpkammervolumen beträgt maximal 1:2, vorzugsweise maximal 1:3, und/oder
c. der Spender (10) weist stromaufwärts der Abgabeöffnung (28) ein Abgabeventil (26) auf, wobei das Abgabeventil vorzugsweise zum Öffnen ab einem Überdruck von 0,3 bar ausgebildet ist, und/oder
d. der Flüssigkeitsspeicher (14) des Spenders weist eine Mantelfläche (15) auf, die einstückig mit dem die Pumpeinrichtung (16) umgebenden Gehäuse ausgebildet, wobei vorzugsweise an einem der Abgabeöffnung (28) gegenüberliegenden Ende ein als separates Teil ausgebildeter Boden (12) an der Mantelfläche (15) befestigt ist, und/oder
e. der Flüssigkeitsspeicher ist mittels eines Belüftungskanals mit einer umgebenden Atmosphäre verbunden oder der Flüssigkeitsspeicher weist ein variables Innenvolumen auf und ist hierfür durch eine flexible oder verlagerbare Wandung begrenzt.

## Claims

1. Dispenser (10) for discharge of pharmaceutical liquids, especially for the dropwise discharge of pharmaceutical liquids in the form of individual droplets, having the following features:
a. the dispenser (10) has a liquid reservoir (14), a pump device (16) having a volumetrically variable pump chamber (20), and a delivery opening (28) for delivery of the liquid, and
b. the dispenser (10) has an elongate housing aligned in a main direction of extension (2), wherein the delivery opening (28) is provided at a distal end of the dispenser (10), and
c. the dispenser (10) has an actuating button (70) which is provided in the region of an outer surface (54) of the housing at the side of the dispenser (10) and can be depressed between an unactuated starting position and an actuated final position, and
d. the volumetrically variable pump chamber (20) is bounded on one side by a displaceable and/or deformable pump chamber wall (64) connected to the actuating button (70) in a connecting region (70A) in such a way that it reduces the size of the pump chamber through depression of the actuating button (70), and
e. the dispenser (10) has a spring device (72) that acts on the actuating button (70) in a force input region (70B) and by which the actuating button (70) is subjected to a force in the direction of its unactuated starting position, and
f. the actuating button (70) takes the form of a rocker button (70) designed to be rockable with respect to the housing by rocking about a rocker axis (6),
**characterized by** the following feature:
g. the force input region (70B) and the connecting region (70A) are provided on the rocker button (70) at different distances from the rocker axis (6).

2. Dispenser (10) according to Claim 1, having the following further feature:
a. the rocker axis (6) is provided at an opposite end of the rocker button (70) from the delivery opening (28) based on the main direction of extension (2).

3. Dispenser (10) according to Claim 1 or 2, having the following further feature:
a. a detent (37) is provided at the remote side of the rocker button (70) from the rocker axis (6), makes it difficult to depress the rocker button (70) and preferably requires an actuating force of at least 10 newtons at the remote end of the rocker button (70) from the rocker axis (6) to overcome it.

4. Dispenser (10) according to any of the preceding claims, having at least one of the following additional features:
b. the force input region (70B) is further removed from the rocker axis (6) than the connecting region (70A) and/or
c. the distance between the rocker axis (6) and the force input region (70B) and the distance between the rocker axis (6) and the connecting region (70A) differ from one another by at least 20% based on the smaller of the distances.

5. Dispenser (10) according to any of the preceding claims, having at least one of the following further features:
a. an elastically deformable compensation section (66) is provided between the rocker button (70) and the displaceable and/or deformable pump chamber wall (64), and/or
b. the pump chamber wall (64) is formed by a deformable membrane portion (60).

6. Dispenser (10) according to any of the preceding claims, having the following further feature:
a. at least one axis section (74) accommodated in a bearing (40) on the housing side is provided in the rocker button (70).

7. Dispenser (10) according to any of the preceding claims, having the following further features:
a. the dispenser (10) has a channel section (24) which is aligned in main direction of extension (2), which is provided downstream of the pump chamber (20) and through which liquid can be conveyed to the delivery opening (28), and
b. the dispenser (10) has an insert (50) inserted into the channel section (24) from a distal end in order to reduce its free volume.

8. Dispenser (10) according to Claim 7, having the following further features:
a. the dispenser (10) has a first housing component (30) in which the channel section (24) is provided, and
b. the dispenser (10) has a second housing component (44) which is disposed at the distal end of the first housing component (30) and which is penetrated by the delivery opening (28), and
c. the insert (50) is provided in the second housing component (44).

9. Dispenser (10) according to Claim 8, having the following further features:
a. the first housing component (30) has a first circumferential land (32) at its distal end, and
b. the second housing component (44) has a second circumferential land (48), and
c. a sealing surface on the outside of the second circumferential land (48) adjoins an inner face of the first circumferential land (32) to form a circumferential seal region,
especially having the following additional feature:
d. the insert (50) is provided as an appendage on the second circumferential land (48).

10. Dispenser (10) according to any of the preceding claims, having the following further features:
a. the housing has a first housing component (30) that bounds at least sections of the pump chamber (20), and
b. the housing has a second housing component (44) penetrated by the delivery opening (28), and
c. the housing has an outer housing component (52) that at least partly surrounds the first and second housing components (30, 44), wherein the second housing component (44) projects out of the outer housing component (52) through an aperture (58) therein, and wherein an aperture (56) for actuation of the actuating button (70) is provided, and
d. the outer housing component (52) has no liquid-guiding surfaces at all that bound a liquid pathway between liquid reservoir (14) and delivery opening (28).

11. Dispenser (10) according to Claim 10, having the following further feature:
a. the second housing component (44) is secured, preferably clamped, between a distal end of the outer housing component (52) and the distal end of the first housing component (30).

12. Dispenser (10) according to Claim 10 or 11, having the following further features:
a. the actuating button (70) has a first button component (80),
- which is mounted on the first housing component (30) so as to be movable, and/or
- which has a connecting region (70A) for connection to a displaceable pump chamber wall (64) and/or
- which has a force input region (70B) for introduction of the force from the spring device (72),
and
b. the actuating button (70) has a second button component (76) mounted on the outside of the first button component (80) and has a pressure surface (78) for direct manual application of force.

13. Dispenser (10) according to any of the preceding claims, having the following further feature:
a. the dispenser (10) is designed as a droplet dispenser (10) and has means of droplet formation downstream of the delivery opening (28),
preferably having at least one of the following additional features:
b. the means of droplet formation comprise a droplet formation surface which surrounds the delivery opening and is preferably in flat or concave form and/or which is preferably surrounded by a sharp break-off edge, and/or
c. the dispenser has a coordination between the pump device (16) and the means of droplet formation by way of which a complete actuation of the actuating button produces exactly one falling droplet.

14. Dispenser (10) according to any of the preceding claims, having the following further feature:
a. the dispenser (10) has a dispenser cap (100) that can be placed onto the housing and protects the delivery opening when in place,
preferably having at least one of the following additional features:
b. the dispenser cap (100) is configured as a ventilated cap and has a ventilation opening (102) through which the delivery opening (28) is or can be connected to a surrounding atmosphere even with the dispenser cap (100) in place, preferably with a sterile filter (104) assigned to the ventilation opening (102), and/or
c. the dispenser cap (100) has a pad (106) which, with the dispenser cap (100) in place, is positioned over or on the delivery opening (28) such that any liquid residue that remains downstream of the delivery opening (28) is absorbed and/or decontaminated thereby.

15. Dispenser (10) according to any of the preceding claims, having at least one of the following features:
a. the difference between a maximum pump chamber volume and a minimum pump chamber volume is between 20 µl and 50 µl, preferably between 30 µl and 40 µl, and/or
b. the quotient between a minimum pump chamber volume and a maximum pump chamber volume is not more than 1:2, preferably not more than 1:3, and/or
c. the dispenser (10) has a delivery valve (26) upstream of the delivery opening (28), wherein the delivery valve is preferably designed to open over and above a positive pressure of 0.3 bar, and/or
d. the liquid reservoir (14) of the dispenser has an outer surface (15) in one-piece form together with the housing surrounding the pump device (16), wherein a base (12) in the form of a separate part is preferably secured to the outer surface (15) at an opposite end from the delivery opening (28), and/or
e. the liquid reservoir is connected to a surrounding atmosphere by means of a ventilation channel, or the liquid reservoir has a variable internal volume and is bounded for the purpose by a flexible or displaceable wall.

## Revendications

1. Distributeur (10) destiné à la sortie de liquides pharmaceutiques, en particulier pour la sortie goutte à goutte de liquides pharmaceutiques sous forme de gouttes individuelles, présentant les caractéristiques suivantes :
a. le distributeur (10) présente un réservoir de liquide (14), un système de pompe (16) doté d'une chambre de pompe (20) de volume variable ainsi qu'une ouverture de distribution (28) pour la distribution du liquide, et
b. le distributeur (10) présente un boîtier allongé orienté le long d'une direction d'étendue principale (2), l'ouverture de distribution (28) étant prévue à une extrémité distale du distributeur (10), et
c. le distributeur (10) présente un bouton d'actionnement (70) qui est prévu latéralement sur le distributeur (10) dans la région d'une surface d'enveloppe (54) du boîtier et qui peut être enfoncé entre une position initiale non actionnée et une position finale actionnée, et
d. la chambre de pompe (20) de volume variable est limitée sur un côté par une paroi de chambre de pompe (64) déplaçable et/ou déformable qui est reliée au bouton d'actionnement (70) dans une région de liaison (70A) de telle sorte qu'elle réduit la chambre de pompe par enfoncement du bouton d'actionnement (70), et
e. le distributeur (10) présente un dispositif ressort (72) qui agit sur le bouton d'actionnement (70) dans une région d'introduction de force (70B) et par le biais duquel le bouton d'actionnement (70) est soumis à une force en direction de sa position initiale non actionnée, et
f. le bouton d'actionnement (70) est réalisé sous forme de bouton basculant (70) qui est réalisé de manière mobile en basculement autour d'un axe de basculement (6) par rapport au boîtier,
**caractérisé par** la caractéristique suivante :
g. la région d'introduction de force (70B) et la région de liaison (70A) sont prévues sur le bouton basculant (70) de manière éloignée à des distances différentes de l'axe de basculement (6).

2. Distributeur (10) selon la revendication 1 présentant l'autre caractéristique suivante :
a. l'axe de basculement (6) est prévu à une extrémité du bouton basculant (70) opposée à l'ouverture de distribution (28) par rapport à la direction d'étendue principale (2).

3. Distributeur (10) selon la revendication 1 ou 2 présentant l'autre caractéristique suivante :
a. un cran (37) est prévu sur le côté du bouton basculant (70) opposé à l'axe de basculement (6), lequel cran rend difficile un enfoncement du bouton basculant (70) et nécessite de préférence une force d'actionnement d'au moins 10 Newton à l'extrémité du bouton basculant (70) opposée à l'axe de basculement (6) pour être surmonté.

4. Distributeur (10) selon l'une des revendications précédentes présentant au moins l'une des caractéristiques supplémentaires suivantes :
b. la région d'introduction de force (70B) est plus éloignée de l'axe de basculement (6) que la région de liaison (70A) et/ou
c. la distance entre l'axe de basculement (6) et la région d'introduction de force (70B) et la distance entre l'axe de basculement (6) et la région de liaison (70A) diffèrent l'une de l'autre d'au moins 20% par rapport à la plus petite des distances.

5. Distributeur (10) selon l'une des revendications précédentes présentant au moins l'une des autres caractéristiques suivantes :
a. une partie de compensation (66) déformable élastiquement est prévue entre le bouton basculant (70) et la paroi de chambre de pompe (64) déplaçable et/ou déformable, et/ou
b. la paroi de chambre de pompe (64) est formée par une partie de membrane (60) déformable.

6. Distributeur (10) selon l'une des revendications précédentes présentant l'autre caractéristique suivante :
a. au moins une partie axiale (74) est prévue sur le bouton basculant (70), laquelle partie axiale est reçue dans un support (40) côté boîtier.

7. Distributeur (10) selon l'une des revendications précédentes présentant les autres caractéristiques suivantes :
a. le distributeur (10) présente une partie de canal (24) orientée dans la direction d'étendue principale (2), laquelle partie de canal est prévue en aval de la chambre de pompe (20) et à travers laquelle du liquide peut être refoulé jusqu'à l'ouverture de distribution (28), et
b. le distributeur (10) présente un insert (50) qui est inséré dans la partie de canal (24) depuis une extrémité distale, afin de réduire son volume libre.

8. Distributeur (10) selon la revendication 7 présentant les autres caractéristiques suivantes :
a. le distributeur (10) présente un premier composant de boîtier (30) dans lequel la partie de canal (24) est prévue, et
b. le distributeur (10) présente un deuxième composant de boîtier (44) qui est disposé à l'extrémité distale du premier composant de boîtier (30) et qui est traversé par l'ouverture de distribution (28), et
c. l'insert (50) est prévu sur le deuxième composant de boîtier (44).

9. Distributeur (10) selon la revendication 8 présentant les autres caractéristiques suivantes :
a. le premier composant de boîtier (30) comprend à son extrémité distale une première nervure périphérique (32), et
b. le deuxième composant de boîtier (44) présente une deuxième nervure périphérique (48), et
c. une surface d'étanchéité s'appuie contre le côté extérieur de la deuxième nervure périphérique (48) avec formation d'une région d'étanchéité périphérique sur un côté intérieur de la première nervure périphérique (32), en particulier présentant la caractéristique supplémentaire suivante :
d. l'insert (50) est prévu en tant que prolongement sur la deuxième nervure périphérique (48).

10. Distributeur (10) selon l'une des revendications précédentes présentant les autres caractéristiques suivantes :
a. le boîtier présente un premier composant de boîtier (30), lequel limite au moins dans certaines parties la chambre de pompe (20), et
b. le boîtier présente un deuxième composant de boîtier (44), lequel est traversé par l'ouverture de distribution (28), et
c. le boîtier présente un composant de boîtier extérieur (52), lequel entoure au moins partiellement le premier et le deuxième composant de boîtier (30, 44), le deuxième composant de boîtier (44) faisant saillie hors du composant de boîtier extérieur (52) à travers un orifice (58) de celui-ci et un orifice (56) étant prévu pour l'actionnement du bouton d'actionnement (70), et
d. le composant de boîtier extérieur (52) ne présente pas de surfaces acheminant un liquide, qui limitent une voie de liquide entre le réservoir de liquide (14) et l'ouverture de distribution (28).

11. Distributeur (10) selon la revendication 10 présentant l'autre caractéristique suivante :
a. le deuxième composant de boîtier (44) est fixé, de préférence serré, entre une extrémité distale du composant de boîtier extérieur (52) et l'extrémité distale du premier composant de boîtier (30).

12. Distributeur (10) selon la revendication 10 ou 11 présentant les autres caractéristiques suivantes :
a. le bouton d'actionnement (70) présente un premier composant de boîtier (80),
- lequel est monté mobile sur le premier composant de boîtier (30), et/ou
- lequel présente une région de liaison (70A) pour la liaison à une paroi de chambre de pompe (64) déplaçable et/ou
- lequel présente une région d'introduction de force (70B) pour l'application de la force du dispositif ressort (72),
et
b. le bouton d'actionnement (70) présente un deuxième composant de boîtier (76), lequel est attaché au premier composant de boîtier (80) du côté extérieur et présente une surface de pression (78) pour l'application manuelle directe d'une force.

13. Distributeur (10) selon l'une des revendications précédentes présentant l'autre caractéristique suivante :
a. le distributeur (10) est réalisé sous forme de distributeur de gouttes (10) et présente des moyens de formation de gouttes en aval de l'ouverture de distribution (28),
de préférence présentant au moins l'une des caractéristiques supplémentaires suivantes :
b. les moyens de formation de gouttes comprennent une surface de formation de gouttes entourant l'ouverture de distribution, laquelle surface est formée de préférence de manière plane ou concave et/ou laquelle est entourée de préférence par une arête de détachement vive, et/ou
c. le distributeur présente une adaptation entre le dispositif de pompe (16) et les moyens de formation de gouttes, par laquelle un actionnement complet du bouton d'actionnement produit exactement une goutte tombante.

14. Distributeur (10) selon l'une des revendications précédentes présentant l'autre caractéristique suivante :
a. le distributeur (10) présente un capuchon de distributeur (100) qui peut être placé sur le boîtier et protège l'ouverture de distribution dans l'état placé,
de préférence présentant au moins l'une des caractéristiques supplémentaires suivantes :
b. le capuchon de distributeur (100) est configuré sous forme de capuchon aéré et présente une ouverture de ventilation (102) par le biais de laquelle l'ouverture de distribution (28) est reliée ou peut être reliée à une atmosphère environnante même lorsque le capuchon de distributeur (100) est placé, un filtre stérile (104) étant de préférence associé à l'ouverture de ventilation (102), et/ou
c. le capuchon de distributeur (100) présente un tampon (106), lequel, lorsque le capuchon de distributeur (100) est placé, est positionné au-dessus ou au niveau de l'ouverture de distribution (28) de telle sorte qu'un reste de liquide demeurant en aval de l'ouverture de distribution (28) soit reçu par celui-ci et/ou décontaminé.

15. Distributeur (10) selon l'une des revendications précédentes présentant au moins l'une des caractéristiques suivantes:
a. la différence entre un volume maximal de chambre de pompe et un volume minimal de chambre de pompe vaut entre 20 µl et 50 µl, de préférence entre 30 µl et 40 µl, et/ou
b. le quotient entre un volume minimal de chambre de pompe et un volume maximal de chambre de pompe vaut au maximum 1:2, de préférence au maximum 1:3, et/ou
c. le distributeur (10) présente une soupape de distribution (26) en amont de l'ouverture de distribution (28), la soupape de distribution étant réalisée de préférence pour s'ouvrir à partir d'une surpression de 0,3 bar, et/ou
d. le réservoir de liquide (14) du distributeur présente une surface d'enveloppe (15) qui est réalisée d'une seule pièce avec le boîtier entourant le dispositif de pompe (16), de préférence un fond (12) réalisé comme partie séparée étant fixé à la surface d'enveloppe (15) à une extrémité opposée à l'ouverture de distribution (28), et/ou
e. le réservoir de liquide est relié à une atmosphère environnante au moyen d'un canal d'aération ou le réservoir de liquide présente un volume intérieur variable et est à cet effet limité par une paroi flexible ou déplaçable.
